Europäisches Patentamt

European Patent Office

Office européen des brevets

(11) Numéro de publication: **0 322 493 B1**

(12) **FASCICULE DE BREVET EUROPEEN**

(45) Date de publication de fascicule du brevet:
**14.08.91**

(51) Int. Cl.⁵: **A61F 2/32**, A61F 2/42

(21) Numéro de dépôt: **87402994.5**

(22) Date de dépôt: **28.12.87**

(54) **Prothèse articulaire.**

(43) Date de publication de la demande:
**05.07.89 Bulletin 89/27**

(45) Mention de la délivrance du brevet:
**14.08.91 Bulletin 91/33**

(84) Etats contractants désignés:
**AT BE CH DE ES GB GR IT LI LU NL SE**

(56) Documents cités:
**FR-A- 2 603 476**

(73) Titulaire: **Leclercq, Sylvain**
**1 rue du Régiment de la Chaudière**
**F-14990 Bernières sur Mer(FR)**

Titulaire: **Aubriot, Jacques**
**17 rue de l'Académie**
**F-14000 Caen(FR)**

Titulaire: **Mercier, Vincent**
**6 rue des Cordeliers**
**F-14000 Caen(FR)**

(72) Inventeur: **Leclercq, Sylvain**
**1 rue du Régiment de la Chaudière**
**F-14990 Bernières sur Mer(FR)**
Inventeur: **Aubriot, Jacques**
**17 rue de l'Académie**
**F-14000 Caen(FR)**
Inventeur: **Mercier, Vincent**
**6 rue des Cordeliers**
**F-14000 Caen(FR)**

(74) Mandataire: **Fruchard, Guy et al**
**CABINET BOETTCHER 23, rue la Boétie**
**F-75008 Paris(FR)**

## Description

La présente invention concerne une prothèse articulaire et plus particulièrement, bien que non exclusivement, une prothèse de hanche.

Une articulation est formée d'au moins deux surfaces articulaires mobiles l'une par rapport à l'autre. La hanche par exemple est formée d'une part de l'épiphyse fémorale, sphérique, convexe, et d'autre part du cotyle, sphérique, concave. La nécessité d'une prothèse peut provenir soit de la détérioration d'une des deux surfaces, soit de la détérioration des deux. On distingue dans les prothèses de hanche actuelles, deux types de prothèse : les cupules et les prothèses à appui centro-diaphysaire.

Les cupules ne visent que le remplacement des surfaces articulaires pathologiques. On peut remplacer la surface fémorale et la surface cotyloïdienne ou ne remplacer que l'une de ces surfaces. Ces cupules peuvent être scellées ou non. Ce type de prothèse a l'avantage de préserver le tissu épiphysaire et peut être remplacé dans le cas d'usure ou d'échec par une prothèse du deuxième type. La prothèse est stable car son diamètre est important mais cela suppose en contrepartie une usure plus importante.

Le deuxième type de prothèse comporte une queue prothétique dans la diaphyse fémorale. La queue prend appui sur la corticale diaphysaire. La partie articulaire est une sphère qui peut s'articuler directement avec le cotyle naturel ou avec un cotyle prothétique. Dans ce dernier cas, il est possible et intéressant de diminuer le diamètre de la sphère pour diminuer l'usure de la prothèse mais il existe alors un risque d'instabilité et de luxation. De plus, ces prothèses supposent une resection du stock osseux plus importante que dans le cas des cupules de sorte qu'en cas d'usure ou d'échec elles ne peuvent être remplacées que par des prothèses de même type au cours d'une intervention souvent longue et difficile.

Par ailleurs, l'os est un tissu vivant en perpétuel remaniement. Sa densification ou au contraire sa résorption dépendent de plusieurs facteurs parmi lesquels les contraintes mécaniques sont les plus importantes. Or la mise en place d'une prothèse articulaire va perturber le champ de contraintes transmis dans le tissu osseux.

On distingue schématiquement quatre types de contraintes : le cisaillement, la traction, la flexion et la compression. Les contraintes en compression sont celles qui permettent le mieux d'obtenir une réponse osseuse de bonne qualité à condition qu'elles conservent une valeur proche de la physiologie.

Un but de la présente invention est de créer une prothèse articulaire favorisant une transmission des forces suivant les lignes naturelles et favorisant les forces de compression pour préserver ou reconstituer le stock osseux.

En vue de la réalisation de ce but, on prévoit selon l'invention une prothèse articulaire comportant deux parties de surface opposée convexes sensiblement concentriques, l'une des parties de surface convexes ayant un rayon de courbure supérieur à l'autre.

Ainsi, la direction des forces appliquées sur la prothèse passe par le centre de celle-ci et respecte l'équilibre de l'articulation. De plus, la transmission de la force résultante au niveau de l'interface articulaire se fait selon une direction normale aux parties de surface convexes, ce qui est généralement conforme à la direction des travées naturelles dans les os reliés par l'articulation.

Selon un mode de réalisation avantageux de l'invention, les parties de surface convexes sont des calottes sphériques. Ainsi on obtient un glissement aisé de la prothèse dans les cavités dans lesquelles elle est insérée.

Selon un autre aspect avantageux de l'invention l'une des parties de surface convexes a un rayon de courbure supérieur à l'autre. Ainsi on favorise le glissement sur l'une des parties de l'articulation.

Selon un mode de réalisation préféré de l'invention associée à une prothèse comportant des parties de surface convexes ayant des rayons de courbure différente, les parties de surface convexes sont reliées l'une à l'autre par un tronc de cône. Ainsi on favorise un débattement maximum de l'articulation autour de la partie de surface convexe de plus petit rayon de courbure. Selon encore un autre aspect particulier de l'invention, la prothèse comporte deux éléments rigides réunis par un organe élastique, chacun des éléments rigides portant l'une des surfaces convexes. Ainsi, l'organe élastique forme un amortisseur entre les deux os associés l'un à l'autre par la prothèse.

D'autres caractéristiques et avantages de l'invention apparaîtront à la lecture de la description qui suit en liaison avec les dessins ci-joints, parmi lesquels :

- la figure 1 est une vue en élévation d'un mode de réalisation d'une prothèse selon l'invention,
- la figure 2 est une vue en coupe d'une articulation de hanche équipée d'une prothèse selon l'invention.

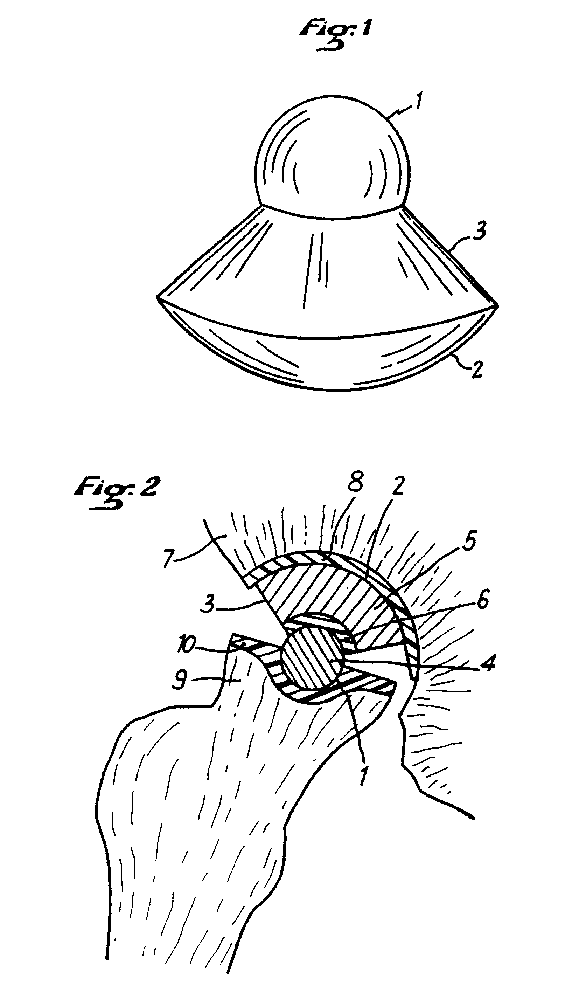

En relation avec les figures, la prothèse articulaire selon l'invention comporte une première partie de surface convexe 1, par exemple une calotte sphérique associée à une seconde partie de surface convexe concentrique opposée 2, par exemple une seconde calotte sphérique ayant un rayon de courbure supérieur à la première calotte sphérique 1, par une surface en tronc de cône 3. Dans le mode de réalisation de la figure 1, la prothèse est formée en une seule pièce, par exemple en acier inoxydable.

La figure 2 illustre une application de la prothèse selon l'invention à une articulation de hanche représentée en coupe selon un plan passant sensiblement par l'axe du fémur. Dans ce mode de réalisation, la première surface convexe 1 est la surface externe d'une bille en acier 4, tandis que la seconde surface convexe opposée 2 est formée par la surface externe d'un tronçon de bille creuse 5 qui est relié à la bille 4 par un organe élastique 6, par exemple une pièce en polyéthylène ou en élastomère collée à la bille 4 et au tronçon 5.

Dans l'application illustrée, l'os du bassin 7 a été légèrement creusé et une cupule 8 a été implantée dans le cotyle fémoral. Par ailleurs, la tête 9 a été sectionnée selon un plan passant par le centre de la tête et normal à l'axe des lignes de force du col fémoral. Le moignon a ensuite été creusé pour mettre en place une cupule fémorale 10.

Le diamètre de la surface de plus grand rayon 2 est de préférence disposé dans la cupule cotyloïdienne tandis que la surface 1 de petit diamètre est de préférence insérée dans la cupule fémorale. Afin de minimiser les forces de frottement, il est préférable que la surface de petit diamètre 1 ait un rayon aussi faible que possible tout en étant toutefois compatible avec les contraintes de pression transmises par la prothèse. De son côté, la surface de grand diamètre doit être de préférence un peu supérieure au diamètre de la tête du fémur afin de permettre un débattement de la prothèse dans la cupule cotyloïdienne 8 dans le cas où la surface supérieure de la cupule fémorale 10 viendrait en contact avec la paroi tronconique 3. Pour une articulation de hanche humaine de 49 mm de diamètre, il est suggéré d'adopter un grand diamètre d'environ 50 mm et un petit diamètre d'environ 22 mm. Les cupules peuvent être réalisées en différents matériaux, dont l'épaisseur dépendra de l'articulation dans laquelle la prothèse est implantée et du matériau choisi. Pour une articulation de hanche, les cupules réalisées en polyéthylène peuvent avantageusement avoir une épaisseur de 6 à 8 mm.

On remarque que dans le mode de réalisation illustré le caractère concentrique de tous les éléments et de toutes les surfaces des différentes pièces permet d'éliminer tout couple mécanique. Il n'existe donc aucun travail en flexion ou en traction au niveau des interfaces d'implantation de la prothèse et une reconstitution du tissu osseux au niveau de l'interface avec les cupules est donc très nettement favorisée.

De plus, toutes les contraintes appliquées à la prothèse sont des forces de compression passant par le centre de la prothèse. L'équilibre statique de la prothèse est donc permanent. La mobilisation se fait naturellement sur le couple de petit diamètre. Le risque de luxation est limité par la mobilité du couple de grand diamètre. Enfin l'implantation de cette prothèse épargne le stock osseux, ce qui permet une reprise en cas d'échec ou d'usure par une prothèse à appui centro-diaphysaire.

Bien entendu, l'invention n'est pas limitée au mode de réalisation décrit et l'on peut y apporter des variantes de réalisation sans sortir du cadre de protection. En particulier, bien que le mode de réalisation particulier illustré comporte deux surfaces convexes sphériques, des surfaces convexes ayant d'autres formes, par exemple une forme parabolique, peuvent être utilisées, les cupules associées ayant bien entendu une forme complémentaire permettant par exemple un roulement avec glissement sur une partie du débattement de la prothèse. De même, bien que le mode de réalisation décrit comporte des surfaces convexes exactement concentriques, on peut également prévoir des surfaces convexes légèrement décentrées en particulier pour des articulations telles que l'épaule. Enfin, si l'état du cotyle le permet, on peut monter la prothèse directement dans le cotyle existant sans prévoir de cotyle prothétique.

De préférence, le centre de la prothèse coïncide avec le centre de la tête de fémur ou, dans le cas d'une autre articulation, avec le centre de l'épiphyse qui est remplacée par la prothèse.

En définitive, la forme exacte de la prothèse, sa dimension et les matériaux à utiliser dépendent de l'articulation concernée et seront adaptés à chaque cas particulier pour tenir compte de la morphologie de chaque individu.

## Revendications

1. Prothèse articulaire comportant deux parties de surface opposées convexes (1,2) sensiblement concentriques, caractérisée en ce que l'une (2) des parties de surface convexes a un rayon de courbure supérieur à l'autre (1).

2. Prothèse articulaire selon la revendication 1, caractérisée en ce que les parties de surface convexes sont reliées l'une à l'autre par un tronçon de cône (3).

3. Prothèse articulaire selon la revendication 1 ou la revendication 2, caractérisée en ce que les parties de surface convexes sont des calottes sphériques.

4. Prothèse articulaire selon l'une des revendications 1 à 3, caractérisée en ce qu'elle comporte deux éléments rigides (4, 5) réunis par un organe élastique (6), chacun des éléments rigides portant l'une des surfaces convexes.

## Claims

1. A joint prosthesis comprising two substantially concentric convex opposite surface portions (1, 2) characterised in that one (2) of the convex surface portions has a greater radius of curvature than the other (1).

2. A joint prosthesis according to claim 1 characterised in that the convex surface portions are connected to each other by a frustoconical portion (3).

3. A joint prosthesis according to claim 1 or claim 2 characterised in that the convex surface portions are portions of a sphere.

4. A joint prosthesis according to one of claims 1 to 3 characterised in that it comprises two rigid elements (4, 5) connected by an elastic member (6), each of the rigid elements carrying one of the convex surfaces.

## Patentansprüche

1. Gelenkprothese mit zwei voneinander abgewandten konvexen, im wesentlichen konzentrischen Flächenabschnitten, dadurch **gekennzeichnet**, daß einer (2) der konvexen Flächenabschnitte einen größeren Krümmungsradius hat als der andere (1).

2. Gelenkprothese nach Anspruch 1, dadurch **gekennzeichnet**, daß die konvexen Flächenabschnitte miteinander über einen Kegelstumpf (3) verbunden sind.

3. Gelenkprothese nach Anspruch 1 oder 2, dadurch **gekennzeichnet**, daß die konvexen Flächenabschnitte Kugelkalotten sind.

4. Gelenkprothese nach einem der Ansprüche 1 bis 3, dadurch **gekennzeichnet**, daß sie zwei starre, über ein elastisches Organ (6) miteinander verbundene Elemente (4, 5) umfaßt, wobei jedes der starren Elemente jeweils eine der konvexen Flächen aufweist.

*Fig. 1*

*Fig. 2*